# EUROPEAN PATENT APPLICATION

(11) **EP 1 988 164 A1**
(43) Date of publication of application: **05.11.2008**
(21) Application number: 07714799.9
(22) Date of filing: 22.02.2007
(51) Int. Cl.: C12N 15/09, A61K 31/517, A61K 31/5377, A61K 45/00, A61P 43/00, C07D 239/94, C07D 405/04, C07K 16/28, C12Q 1/68, G01N 33/574

(54) **METHOD OF TESTING SENSITIVITY OF SOLID CANCER AGAINST TYROSINE KINASE INHIBITOR AND TEST KIT THEREFOR**

(30) Priority: 23.02.2006 JP 2006046779
(71) Applicant: National University Corporation Kanazawa University, Kanazawa-shi, Ishikawa 920-1164 (JP)
(72) Inventor: KASAHARA, Kazuo, Kanazawa-shi, Ishikawa 920-1164 (JP); NISHIO, Kazuto, Tokyo 104-0045 (JP); KIMURA, Hideharu, Tokyo 104-0045 (JP); TAMURA, Tomohide, Tokyo 104-0045 (JP)
(74) Representative: Denison, Christopher Marcus
(86) International application number: PCT/JP2007/053301
(87) International publication number: WO 2007/099852

(57) **Abstract**

This invention provides a method for testing the sensitivity of solid cancer to tyrosine kinase inhibitors such as gefitinib as a typical example and a test kit therefor, with the use of specimens that can be conveniently collected, such as blood and normal tissue. Specifically, the expression level of an HLA-DQA1 gene or an HLA-DQα1 antigen contained in a sample of a cancer patient is measured and then the sensitivity is tested according to the expression level.

## Description

### Technical Field

The present invention relates to a method for testing the sensitivity of solid cancer to a tyrosine kinase inhibitor and a test kit therefor.

### Background Art

Lung cancer is the leading cause of death due to malignant tumor on an organ-by-organ basis in Japan. The number of deaths from lung cancer is increasing yearly. Non-small-cell lung cancer cases account for about 90% of lung cancer cases. In regards to therapeutic methods, only roughly one-third of cases are surgically resectable cases with the remaining two-thirds of cases are found to be locally-advanced or advanced metastatic lung cancer. Needless to say, early detection and early treatment are important. Even with recent advances in imaging diagnostic technology, it cannot be said that the proportion of early lung cancer cases discovered has increased. Thus, the necessity to make progress in the treatment of advanced lung cancer is increasing. Conventional cytotoxic anticancer agents possess clear efficacy by prolonging life and combating metastatic lung cancer, as well as ameliorating symptoms from lung cancer; however, the results thereof have not advanced. Thus, new therapeutic strategies are required.

Gefitinib (Iressa^{™}) was developed as an inhibitor against the epidermal growth factor receptor (EGFR). Gefitinib was marketed in 2002 in Japan for the first time in the world as a lung cancer drug having a new mechanism of action. Gefitinib can exert dramatic effects against specific cases, but it can cause drug-induced acute lung injury in some cases. It is still vivid in our memory that Gefitinib has become a social problem. Furthermore, Gefitinib has been reported to have high usability for 1) women, 2) adenocarcinoma cases, 3) nonsmokers, and 4) Asians, including Japanese people.

Tyrosine kinase inhibitors other than gefitinib, particularly epidermal growth factor receptor (EGFR) inhibitors, have problems similar to those of gefitinib.

Therefore, appropriate selection of cases is required for effective clinical application of tyrosine kinase inhibitors. EGFR gene mutation in lung cancer tumor tissue has been reported to date as a factor for defining the antitumor effects of gefitinib (N Engl J Med. 2004; 350(21): 2129-2139). Furthermore, it has been suggested that cases of tumor cells overexpressing the EGFR gene are also important factors for defining the antitumor effects of EGFR inhibitors, including gefitinib (J Natl Cancer Inst. 2005; 97(9): 643-655).

However, it is not easy to collect tumor tissue sufficiently resistant to such genetic testing in advanced non-small-cell lung cancer cases. The present inventors have also attempted to analyze such cases but have actually analyzed only about 40% thereof.

### Disclosure of the Invention

The objective of the present invention is to provide a method for testing the sensitivity of solid cancer to tyrosine kinase inhibitors such as gefitinib as a typical example and a test kit to be used on specimens that can be conveniently collected, such as blood and normal tissue.

The present invention is as summarized as follows.
(1) A method for testing the sensitivity of solid cancer to a tyrosine kinase inhibitor, wherein the sensitivity is tested using an HLA-DQA1 gene or an HLA-DQα1 antigen contained in a sample from a subject.
(2) The method according to (1) above, which comprises measuring the expression level of the HLA-DQA1 gene or the HLA-DQα1 antigen contained in the sample from the subject.
(3) The method according to (1) or (2) above, wherein the tyrosine kinase inhibitor is an epidermal growth factor receptor inhibitor.
(4) The method according to (3) above, wherein the epidermal growth factor receptor inhibitor is gefitinib (Iressa^{™}), erlotinib (Tarceva^{™}), ZD6474 (Zactima^{™}), or lapatinib.
(5) The method according to any one of (1) to (4) above, wherein the sample from the subject is a blood sample.
(6) The method according to (5) above, wherein the blood sample is a peripheral blood mononuclear cell separated from the blood from the subject.
(7) The method according to any one of (1) to (6) above, wherein the solid cancer is lung cancer.
(8) The method according to (2) above, wherein the expression level of the HLA-DQA1 gene is measured using oligonucleotide primers that are at least 15 continuous nucleotides in length or a polynucleotide probe that is at least 15 continuous nucleotides in length and that hybridizes specifically to the HLA-DQA1 gene for specific amplification of the HLA-DQA1 gene.
(9) A test kit for testing the sensitivity of solid cancer to a tyrosine kinase inhibitor, containing oligonucleotide primers that are at least 15 continuous nucleotides in length or a polynucleotide probe that is at least 15 continuous nucleotides in length and hybridizes specifically to an HLA-DQA1 gene for specific amplification of the HLA-DQA1 gene.
(10) An antibody against an HLA-DQα1 antigen, which is used in the method according to (2) above.
(11) A test kit for testing the sensitivity of solid cancer to a tyrosine kinase inhibitor, which contains the antibody of (10) above.
(12) A solid cancer therapeutic agent, which comprises a tyrosine kinase inhibitor and is applied to a cancer patient with solid cancer determined to have sensitivity to the tyrosine kinase inhibitor by the method according to any one of (1) to (8) above.

### Effects of the Invention

According to the present invention, a method for testing the sensitivity of solid cancer to a tyrosine kinase inhibitor and a test kit can be provided; and an appropriate solid cancer therapy can be selected.

### Best Mode for Carrying Out the Invention

Examples of solid cancer targeted in the present invention include, but are not particularly limited to, lung cancer (e.g, adenocarcinoma, squamous cell cancer, and large cell carcinoma), breast cancer, head and neck cancer, mesothelioma, large bowel cancer, and ovarian cancer.

Examples of the HLA gene of the present invention include DNA encoding HLA and mRNA transcribed from the DNA.

Solid cancer can be diagnosed by a general method. For example, lung cancer is diagnosed by diagnostic imaging (computed tomography, CT), positron emission tomography (PET), sputum cytology, transbronchial lung biopsy, percutaneous lung biopsy, or the like.

Among solid cancer cases subjected to administration of tyrosine kinase inhibitors such as gefitinib as a typical example, the present inventors have discovered that the expression level of the HLA-DQA1 gene contained in peripheral blood mononuclear cells is significantly higher in cases successful in terms treatment (partial response (PR)) than in cases in which no changes are apparent after administration or in cases in which exacerbations are apparent (stable disease (SD) and progressive disease (PD)).

Therefore, the expression level of the HLA-DQα1 antigen or the HLA-DQA1 gene in a sample from a subject is measured and a subject exhibiting a high expression level thereof is treated via administration of a tyrosine kinase inhibitor, whereas a subject exhibiting a low expression level thereof is treated by another therapy, such as treatment with an anticancer agent having cytotoxicity (e.g., cisplatin). In this manner, more appropriate solid cancer treatment is made possible.

Examples of the above subject include solid cancer patients, patients with precancerous conditions, and patients suspected of having cancer based on subjective symptoms or various tests.

Examples of the above samples that can be used herein include blood samples such as peripheral blood mononuclear cells separated from blood and samples separated from normal tissue such as oral mucosa and hair.

Examples of techniques for analyzing and measuring the HLA-DQα1 antigen or the HLA-DQA1 gene include an ELISA method, a Western blot method, a bioassay, a radioimmunoassay, an enzyme immunoassay, an immunoagglutination method, a fluorescent antibody method, an electrophoresis method, and genetic analysis using a DNA microarray method or a quantitative RT (reverse transcriptase)-PCR method and preferably genetic analysis using a DNA microarray method. Furthermore, examples of an antibody for measuring the HLA-DQα1 antigen include an antibody that specifically recognizes the HLA-DQα1 antigen and a combination of an antibody that recognizes HLA-DQ entirely and an antibody that does not recognize the HLA-DQα1 antigen.

An example of techniques for measuring the expression level of the HLA-DQA1 gene is preferably a method that involves measuring the expression level of the HLA-DQA1 gene using oligonucleotide primers that are at least 15 continuous nucleotides in length or a polynucleotide probe that is at least 15 continuous nucleotides in length and hybridizes specifically to the HLA-DQA1 gene for specific amplification of the HLA-DQA1 gene.

Units and measured values of the expression level of the HLA-DQα1 antigen or the HLA-DQA1 gene differ depending on employed measurement methods. With the use of any method, data of generally 10 or more and preferably 20 or more solid cancer cases subjected to administration of a tyrosine kinase inhibitor such as gefitinib as a typical example are collected. Furthermore, the cut-off value of the expression level is determined so that the success rate is 80%, for example, based on the correlation between the effect of a tyrosine kinase inhibitor in each case and the expression level of the HLA-DQα1 antigen or the HLA-DQA1 gene. A patient showing an expression level higher than the cut-off value is treated by administration of the tyrosine kinase inhibitor and a patient showing an expression level lower than the cut-off value is treated by another therapy, making it possible to perform effective solid cancer treatment. The number of subjects to be treated using the tyrosine kinase inhibitor decreases as the above cut-off value is increased; however, the efficacy of treatment using the tyrosine kinase inhibitor is increased. The cut-off value is preferably determined for every cancer of the same type. Moreover, it is preferable to determine the cut-off value after measurement is performed so as to lower the bias in view of background factors, individual differences, and the like with regard to solid cancer cases.

For example, when an expression value of the HLA-DQA1 gene measured with the use of a DNA microarray is used as an indicator, the expression value of 3,000 is determined to be the cut-off value, so that a patient showing an expression value of 3,000 or higher is treated by administration of a tyrosine kinase inhibitor. A patient showing an expression value of less than 3,000 is treated by another therapy. This makes it possible to treat lung cancer more effectively.

Furthermore, among lung cancer cases subjected to administration of a tyrosine kinase inhibitor such as gefitinib as a typical example, the expression levels of C2orf33, GLS, KIAA1117, ALDH6A1, PDCD5, and DKFZp761A078 contained in peripheral blood mononuclear cells tend to be lower in cases (partial response (PR)) of successful treatment, in contrast to that of the HLA-DQA1 gene, compared with cases in which no changes are apparent and cases in which exacerbations are apparent (stable disease (SD) and progressive disease (PD)). Accuracy can be further improved when the expression levels of these genes are also used as indicators.

Examples of a tyrosine kinase inhibitor targeted in the present invention include, preferably epidermal growth factor receptor inhibitors such as gefitinib (Iressa^{™}), erlotinib (Tarceva^{™}), ZD6474 (Zactima^{™}), and lapatinib.

Usages and doses of these tyrosine kinase inhibitors may be employed according to their general usages and doses. For example, in the case of gefitinib (Iressa^{™}), generally, 250 mg of gefitinib is orally administered to an adult once a day.

The testing method of the present invention is performed before administration of a tyrosine kinase inhibitor. As a result, only cancer patients with solid cancer determined to have sensitivity to the tyrosine kinase inhibitor are subjected to treatment using the tyrosine kinase inhibitor, so that efficacy can be increased and the incidence of side effects can be decreased.

Moreover, a recommendation is indicated in product instructions such as an attached document for a tyrosine kinase inhibitor, such that the testing method of the present invention is performed before administration, so that the tyrosine kinase inhibitor is administered to only cancer patients with solid cancer determined to have sensitivity. Thus, the efficacy is increased and the incidence of side effects can be decreased.

When the expression level of the HLA-DQα1 antigen is quantified using the ELISA method or the Western blot method, a test kit containing an antibody against the HLA-DQα1 antigen is used.

The above immunoassay kit is composed of, for example, a reagent such as a buffer (labeled antibody solution) containing a labeled antibody, an immobilized antibody, and a reference material. Furthermore, according to need, the kit is composed of a buffer for causing a reaction of a specimen with an immobilized antibody via a sandwich method, a chromogenic solution for an enzymatic reaction, a stopping solution, a cleaning solution for cleaning the solid phase, a pretreatment agent for a specimen, and the like. When such a component reagent is a freeze-dried product, a solution for restoration may also be contained.

When such a kit is based on the solid phase method, types and shapes of solid phases are not limited and may be used for general known immunoassay kits. Examples of such solid phases include plastic test tubes, polystyrene beads, polystyrene particles, polystyrene microplates, polyethylene terephthalate microplates, polyvinyl chloride microplates, magnetic particles, glass beads, cellulose particles, nitrocellulose membranes, cellulose filter paper, and nylon membranes. Test specimens of a convenient assay kit based on the principle of immunochromatography or the like can also be used herein.

An example of the test kit for testing the sensitivity of solid cancer to tyrosine kinase inhibitors, wherein the sensitivity is tested with the use of the HLA-DQA1 gene contained in samples of cancer patients, is a kit containing a primer pair specific to the HLA-DQA1 gene and a reagent thought to be necessary for gene amplification.

The above test kit preferably contains oligonucleotide primers that are at least 15 continuous nucleotides in length or a polynucleotide probe that is at least 15 continuous nucleotides in length and hybridizes specifically to the HLA-DQA1 gene. Such oligonucleotide primers that are at least 15 continuous nucleotides in length can be selected from among those shown in SEQ ID NOS: 1 to 11, for example. Examples of such polynucleotide probe that is at least 15 continuous nucleotides in length and hybridizes specifically to the HLA-DQA1 gene include those shown in SEQ ID NOS: 1 to 11.

### Brief Description of the Drawings

Fig. 1 shows the different mean expression values for the relevant genes, depending on the antitumor effects.
Fig. 2 shows different expression values of HLA-DQA1, depending on the antitumor effects.
Fig. 3 shows the results of analyzing survival at the HLA-DQA1 level.

### Examples

The present invention is hereafter described in greater detail with reference to examples. These examples, however, are not intended to limit the scope of the present invention.

### (Example 1)

### 1. Cases and Methods

Non-small-cell lung cancer cases subjected to administration of gefitinib at the Department of Respiratory Medicine, Kanazawa University Hospital, were examined herein. Cases examined herein are cases of subjects who had received explanations of disease names, other therapies, prognosis, and the like and then agreed to be subjected to examination using peripheral blood mononuclear cells, prior to treatment.

Immediately after blood collection before treatment, peripheral blood mononuclear cells were separated by a specific gravity centrifugation method. RNA was extracted from the thus separated mononuclear cells according to a known method (T-L. Fong et al., J. Clin. Invest., 88, 1058, 1991) and then DNA was synthesized using reverse transcriptase. The DNA (that is, approximately 55,000 genes) was exhaustively analyzed using DNA microarray (produced by Affymetrix, HG-U133 Plus 2.0). Twenty-four (24) analyzable cases were classified into cases successful in terms of treatment (partial response (PR)), cases in which no changes had been apparent, and cases in which exacerbations had been apparent (stable disease (SD) and progressive disease (PD)). They were then subjected to t-test statistical analysis. The significance level was determined to be less than 0.001.

### 2. Results

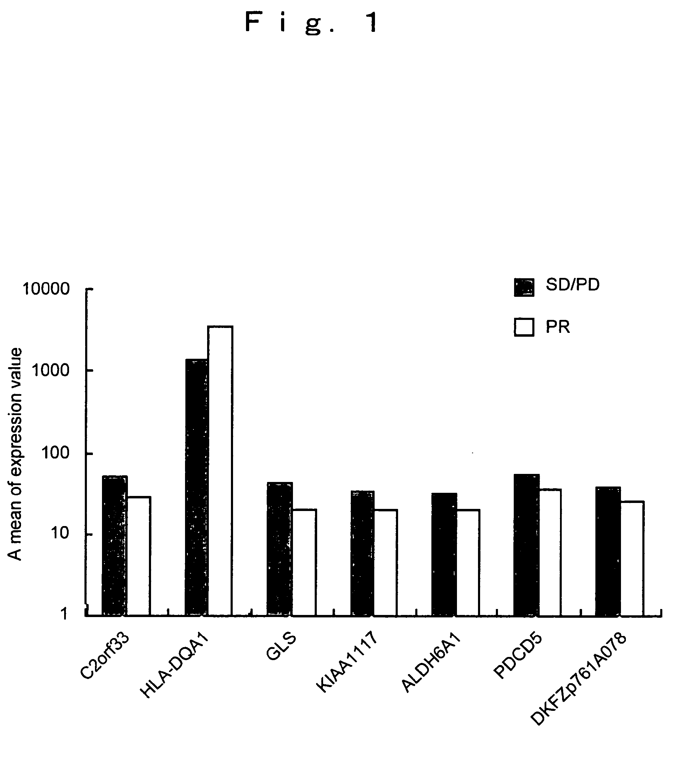

1) Eight (8) of the 24 analyzed cases were determined to be PR cases, and 16 of the same were determined to be SD and PD (SD/PD) cases. The correlations between the resulting expression values as revealed by exhaustive genetic analysis and antitumor effects were analyzed. As a result, the presence of such correlations was confirmed between the expression values of 7 genes and the antitumor effects (Table 1, Fig. 1). The expression levels of the genes other than the HLA-DQA1 gene among the 7 genes were low and determined to be the same or lower than the detection limit of the DNA microarray. Hence, the HLA-DQA1 gene was focused upon and then further analyzed.

**Table 1**

| Genes associated with anti-tumor effects | | | | | | |
|---|---|---|---|---|---|---|
| Rank | Symbol | p-value | Mean expression value | | Ratio* | Description |
| | | | SD/PD | PR | | |
| 1 | C2orf33 | 0.00024 | 48.6 | 28.8 | 1.688 | Chromosome 2 open reading |
| 2 | HLA-DQA1 | 0.00032 | 1374.6 | 3493.3 | 0.393 | frame 33 Major histocompatibility |
| 3 | GLS | 0.00049 | 40.5 | 19.5 | 2.077 | complex, class II, DQ alpha 1 Glutaminase |
| 4 | KIAA1117 | 0.00051 | 32.8 | 19.5 | 1.682 | KIAA1117 |
| 5 | ALDH6A1 | 0.00064 | 31.7 | 19.6 | 1.617 | Aldehyde dehydrogenase 6 family, member A1 |
| 6 | PDCD5 | 0.00072 | 52.9 | 35.4 | 1.494 | Programmed cell death 5 |
| 7 | DKFZp761A078 | 0.00084 | 38.2 | 24.4 | 1.579 | Hypothetical protein DKFZp761A078 |
| 8 | HLA-DQA2 | 0.7863 | 4668.7 | 4204.6 | 1.110 | Major histocompatibility complex, class II, DQ alpha 2 |
| 9 | HLA-DQB1 | 0.1932 | 2831.4 | 1276.3 | 2.218 | Major histocompatibility complex, class II, DQ beta 1 |
| 10 | HLA-DRA1 | 0.1925 | 14441.6 | 11544.4 | 1.251 1 | Major histocompatibility complex, class II, DR alpha 1 |
| 11 | HLA-DRB4 | 0.8734 | 8197.9 | 7747.5 | 1.058 | Major histocompatibility complex, class II, DR beta 4 |

| | | | | | | |
|---|---|---|---|---|---|---|
| * [SD/PD]/PR | | | | | | |

### 2) Examination of the HLA-DQA1 gene

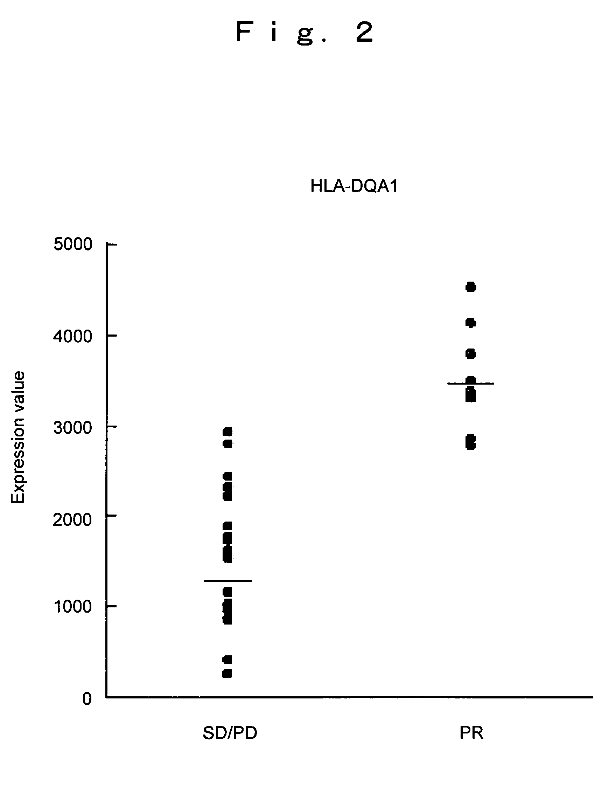

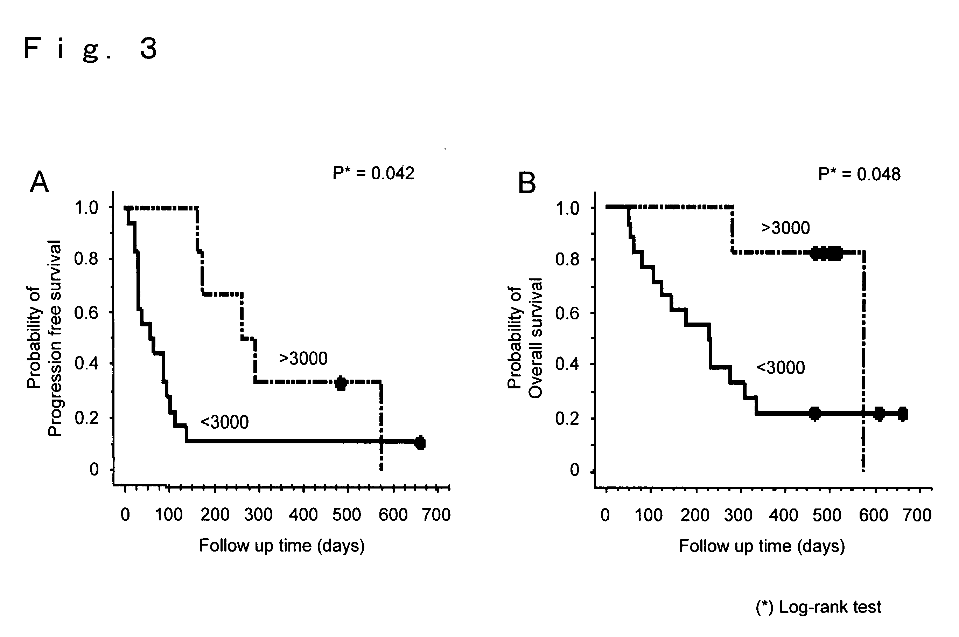

The mean expression value of the HLA-DQA1 gene (the expression levels were high) was 1374.6 (259.0 to 2928.6) in the SD/PD group and 3493.3 (2797.7 to 4532.2) in the PR group (Table 1, Fig. 2). Based on the results, an expression value of 3,000 was determined to be the cut-off value and then the 2 groups were separately examined. In this case, the sensitivity of PR was 75% and the specificity of the same was 100% (Table 2). When survival analysis was performed for two groups (a group with expression values of 3,000 or higher and a group with expression values of less than 3,000), both recurrence-free survival time (Fig. 3A) and overall survival time (Fig. 3B) were found to be significantly prolonged in the group with the expression values of 3,000 or higher.

Based on these results, it was concluded that the HLA-DQA1 expression level could be a predictive factor for the effectiveness of gefitinib against lung cancer.

**Table 2**

| HLA-DQA1 expression and anti-tumor effects | | |
|---|---|---|
| | PR | SD/PD |
| HLA-DQA1 expression | | |
| >3000 | 6 | 0 |
| <3000 | 2 | 16 |

### 3. Consideration

At the present time, no sensitivity predictive factors for patients to be subjected to administration of gefitinib have been established. As described above, EGFR gene mutation or EGFR gene amplification is likely to be a sensitivity predictive factor (factor for predicting sensitivity); however, in actual clinical practice, maneuvers required therefor are too complicated, and thus it can never be used in connection with a standard technique. Furthermore, it is difficult to collect lung cancer tumor tissue. In advanced lung cancer cases, tissue collection is performed for diagnosis using a bronchoscope. The amount of collectable tissue is extremely small (1 mm to 3 mm in size). Since the collected tissue contains stroma other than tumor (blood vessels and supporting tissue), tumor tissue analysis is currently extremely difficult. Furthermore, it is not uncommon for it to be impossible to collect even such tissue. This examination performed by the present inventors using peripheral blood mononuclear cells has merit in that a sample can be collected from any site without requiring any special facility.

There are no reports concerning HLA-DQA1 and gefitinib sensitivity. There are not many reports concerning HLA gene expression and malignant tumors. We believe that association with the HLA gene is interesting in view of the high level of effectiveness of gefitinib on Asians. The fact that sensitivity can be predicted before administration makes it possible to select patients suitable for gefitinib administration, leading to the safe and effective implementation of treatment with gefitinib. Since peripheral blood is used in this method, specimens can be safely and easily collected with less impact on patients subjected to such collection. Furthermore, the method is also applicable to prediction of the therapeutic effects of small-molecule compounds (tyrosine kinase inhibitors, and in particular, epidermal growth factor receptor (EGFR) inhibitors) having similar mechanisms of action, such as erlotinib (Tarceva^{™}), ZD6474 (Zactima^{™}), and lapatinib. With such high expression levels of the HLA-DQA1 gene, analysis at the gene product (HLA-DQα1 antigen) level is also possible.

All publications, patents, and patent applications cited herein are incorporated herein by reference in their entirety.

### Industrial Applicability

The present invention is useful for a method for testing the sensitivity of solid cancer to a remedy for cancer and a test kit therefor.

## Claims

1. A method for testing the sensitivity of solid cancer to a tyrosine kinase inhibitor, wherein the sensitivity is tested using an HLA-DQA1 gene or an HLA-DQα1 antigen contained in a sample from a subject.

2. The method according to claim 1, which comprises measuring the expression level of the HLA-DQA1 gene or the HLA-DQα1 antigen contained in the sample from the subject.

3. The method according to claim 1 or 2, wherein the tyrosine kinase inhibitor is an epidermal growth factor receptor inhibitor.

4. The method according to claim 3, wherein the epidermal growth factor receptor inhibitor is gefitinib (Iressa^{™}), erlotinib (Tarceva^{™}), ZD6474 (Zactima^{™}), or lapatinib.

5. The method according to any one of claims 1 to 4, wherein the sample from the subject is a blood sample.

6. The method according to claim 5, wherein the blood sample is a peripheral blood mononuclear cell separated from the blood from the subject.

7. The method according to any one of claims 1 to 6, wherein the solid cancer is lung cancer.

8. The method according to claim 2, wherein the expression level of the HLA-DQA1 gene is measured using oligonucleotide primers that are at least 15 continuous nucleotides in length or a polynucleotide probe that is at least 15 continuous nucleotides in length and that hybridizes specifically to the HLA-DQA1 gene for specific amplification of the HLA-DQA1 gene.

9. A test kit for testing the sensitivity of solid cancer to a tyrosine kinase inhibitor, containing oligonucleotide primers that are at least 15 continuous nucleotides in length or a polynucleotide probe that is at least 15 continuous nucleotides in length and hybridizes specifically to an HLA-DQA1 gene for specific amplification of the HLA-DQA1 gene.

10. An antibody against an HLA-DQα1 antigen, which is used in the method according to claim 2.

11. A test kit for testing the sensitivity of solid cancer to a tyrosine kinase inhibitor, which contains the antibody of claim 10.

12. A solid cancer therapeutic agent, which comprises a tyrosine kinase inhibitor and is applied to a cancer patient with solid cancer determined to have sensitivity to the tyrosine kinase inhibitor by the method according to any one of claims 1 to 8.


SEQUENCE LISTING

<110> NATIONAL UNIVERSITY CORPORATION KANAZAWA UNIVERSITY
      JAPAN HEALTH SCIENCES FOUNDATION

<120> Method and kit for testing for susceptibility of
      solid cancer to tyrosine kinase inhibitor

<130> PH-2989-PCT

<150> JP 2006-046779
<151> 2006-02-23

<160> 11

<170> PatentIn Ver. 2.1

<210> 1
<211> 25
<212> DNA
<213> Artificial Sequence

<220>
<223> Description of Artificial Sequence: Probe

<400> 1
accaatgagg ttcctgaggt cacag                                        25

<210> 2

<211> 25

<212> DNA

<213> Artificial Sequence

<220>

<223> Description of Artificial Sequence: Probe

<400> 2

atgaggttcc tgaggtcaca gtgtt 25

<210> 3

<211> 25

<212> DNA

<213> Artificial Sequence

<220>

<223> Description of Artificial Sequence: Probe

<400> 3

aggttcctga ggtcacagtg ttttc 25

<210> 4

<211> 25

<212> DNA

<213> Artificial Sequence

<220>
<223> Description of Artificial Sequence: Probe

<400> 4
tcctgaggtc acagtgtttt ccaag 25

<210> 5
<211> 25
<212> DNA
<213> Artificial Sequence

<220>
<223> Description of Artificial Sequence: Probe

<400> 5
gtcttgtgga caacatcttt cctcc 25

<210> 6
<211> 25
<212> DNA
<213> Artificial Sequence

<220>
<223> Description of Artificial Sequence: Probe

<400> 6

ctctccaaga gtgatcattc cttct 25

<210> 7

<211> 25

<212> DNA

<213> Artificial Sequence

<220>

<223> Description of Artificial Sequence: Probe

<400> 7

ccaagagtga tcattccttc ttcaa 25

<210> 8

<211> 25

<212> DNA

<213> Artificial Sequence

<220>

<223> Description of Artificial Sequence: Probe

<400> 8

agagtgatca ttccttcttc aagat 25

<210> 9

<211> 25

<212> DNA

<213> Artificial Sequence

<220>

<223> Description of Artificial Sequence: Probe

<400> 9

agtgatcatt ccttcttcaa gatca 25

<210> 10

<211> 25

<212> DNA

<213> Artificial Sequence

<220>

<223> Description of Artificial Sequence: Probe

<400> 10

gatgagattt atgactgcaa ggtgg 25

<210> 11

<211> 25

<212> DNA

<213> Artificial Sequence

<220>

<223> Description of Artificial Sequence: Probe

<400> 11

cctcttctga aacactggga gcctg 25